# EUROPEAN PATENT APPLICATION

(11) **EP 2 508 890 A1**
(43) Date of publication of application: **10.10.2012**
(21) Application number: 11186504.4
(22) Date of filing: 25.10.2011
(51) Int. Cl.: G01N 33/68, G01N 33/573

(54) **CAIX based diagnosis of heart failure**

(71) Applicant: Roche Diagnostics GmbH, 68305 Mannheim (DE); F. Hoffmann-La Roche AG, 4070 Basel (CH)
(72) Inventor: The designation of the inventor has not yet been filed
(74) Representative: Dick, Alexander

(57) **Abstract**

The present invention is directed to a method of diagnosing heart failure in a subject. The method is based on the determination of the amount of carbonic anhydrase IX (CAIX) in a sample obtained from said subject. Moreover, the invention pertains to a method for assessing whether the myocardium from a subject is protected from necrosis.

## Description

The present invention is directed to a method of diagnosing heart failure in a subject. The method is based on the determination of the amount of carbonic anhydrase IX (CAIX) in a sample obtained from said subject. Moreover, the invention pertains to a method for assessing whether the myocardium from a subject is protected from necrosis.

Carbonic anhydrase IX (CAIX) is a transmembrane protein that consists of an N-terminal signal peptide, an extracellular proteoglycan domain and catalytic domain, a transmembrane segment, and a c-terminal tail. CAIX is upregulated in many types of tumors, such as renal cell carcinoma, non-small lung cancer, breast cancer, and cervical cancer (Liao S.Y et al Cancer Res 1997: 57: 2827; Giatromanolaki A et al, Cancer Res 2001: 61: 7992; Chia S.K. et al J Clin Oncol 2001: 19: 3660; Liao S.Y. et al 1994: 145: 598). Under hypoxic conditions CAIX is induced by its upstream regulator, the hyoxia inducible factor (HIF)-1 transcription factor. CAIX facilitates the tumor cells to create an acidified local environment to promote growth and metastasis (Wykoff C.C. et al Cancer Res 2001: 60: 7075). CAIX has been shown to be associated with poor prognosis in breast cancer (Chen Chi-Long et al Virchows Archiv 2010: 457: 53 - 61), ovarian and renal cell cancer (Pena C et al, Clin Cancer Res 2010: 16: 4853 - 4863) und may also be a target in cancer therapy ( Potter C. et al Cell Cycle 2004: 3: 164 - 167, Swietach P et al, Oncogene 201: 29: 6509 - 6521). Thus CAIX is an important regulator for survival and metastasis in cancer. CAIX can also be measured in the circulation and increased CAIX levels have been found in patients with ovarian and renal cell carcinoma (Carney W.P. Expert Rev Mol Diagn. 2007: 7: 309).

So far CAIX, has not been studied in non-tumor diseases which are associated with hypoxic conditions. One such disease is heart failure.

Heart failure (HF) is a major and growing public health problem. It is estimated that approximately 5 million patients in the USA have HF, more than 500 000 patients are diagnosed with HF for the first time each year, and more than 50.000 patients in the US die each year of HF as a primary cause. Heart failure (HF) is one of the main causes of morbidity and mortality in developed countries. Because of aging of the population and greater longevity of patients with cardiovascular disease incidence and prevalence of HF are increasing. HF is a complex clinical syndrome that impairs the ability of the ventricle to fill with or eject blood and to ensure the body's metabolic needs for supply with blood/oxygen.

The inventors have determined the level of CAIX in patients with heart failure. Advantageously it was shown that CAIX is increased in patients with heart failure when compared to healthy subj ects. Therefore, it was shown by the inventors that CAIX can be used as a marker for diagnosing heart failure. Accordingly, the present invention relates to a method for diagnosing heart failure in a subject based on the determination of the amount of CAIX in said subject.

The inventors further determined the level of a cardiac Troponin, Troponin T, in the heart failure patients (in addition to CAIX). The, thus, determined Troponin levels were correlated to the CAIX levels. Surprisingly, high levels of CAIX were associated with lower levels of Troponin T indicating that CAIX protects from cardiac necrosis in heart failure patients. Accordingly, high levels of CAIX - although unwanted in cancer patients - may be beneficial in heart failure patients.

### Method for diagnosing heart failure

The present invention relates to a method for diagnosing heart failure in a subject comprising a) determining the amount of carbonic anhydrase IX (CAIX) in a sample from the subject.

In a preferred embodiment, the method further comprises the step b) of comparing the, thus, determined amount to a reference amount. Thereby, heart failure is diagnosed in said subj ect.

Accordingly, the present invention, in particular, is directed to a method for diagnosing heart failure in a subject comprising
a) determining the amount of carbonic anhydrase IX (CAIX) in a sample from the subj ect, and
b) comparing the, thus determined amount to a reference amount, whereby heart failure is diagnosed.

Preferably, it is diagnosed whether the subject suffers from heart failure, or not, by carrying out the further step of c) diagnosing whether the subject suffers from heart failure, or not, based on the result of the comparison carried out in step b).

The method of the present invention, preferably, is an ex vivo method. Moreover, it may comprise steps in addition to those explicitly mentioned above. For example, further steps may relate to sample pre-treatments or evaluation of the results obtained by the method. The method may be carried out manually or assisted by automation. Preferably, step (a) and/or (b) may in total or in part be assisted by automation, e.g., by a suitable robotic and sensory equipment for the determination in step (a) or a computer-implemented comparison and/or diagnosis based on said comparison in step (b). More preferably, the method is carried out entirely in an automated manner. In such a case, the diagnostic result which is established in step b) is generated in a suitable output format so that it can be used as an aid for establishing the final clinical diagnosis by, e.g., a medical practitioner.

The term "heart failure" is well known in the art. As used herein, the term, preferably, relates to an impaired systolic and/or diastolic function of the heart being accompanied by overt signs of heart failure. Preferably, heart failure referred to herein is also chronic heart failure.

HF can be classified into various degrees of severity.

According to the NYHA (New York Heart Association) classification, heart failure patients are classified as belonging to NYHA classes I, II, III and IV. A patient having heart failure has already experienced structural and functional changes to his pericardium, myocardium, coronary circulation or cardiac valves. He will not be able to fully restore his health, and is in need of a therapeutical treatment. Patients of NYHA Class I have no obvious symptoms of cardiovascular disease but already have objective evidence of functional impairment. Patients of NYHA class II have slight limitation of physical activity. Patients of NYHA class III show a marked limitation of physical activity. Patients of NYHA class IV are unable to carry out any physical activity without discomfort. They show symptoms of cardiac insufficiency at rest.

This functional classification is supplemented by the more recent classification by the American College of Cardiology and the American Heart Association (see J. Am. Coll. Cardiol. 2001;38;2101-2113, updated in 2005, see J. Am. Coll. Cardiol. 2005;46;el-e82). 4 stages A, B, C and D are defined. Stages A and B are not HF but are considered to help identify patients early before developing "truly" HF. Stages A and B patients are best defined as those with risk factors for the development of HF. For example, patients with coronary artery disease, hypertension, or diabetes mellitus who do not yet demonstrate impaired left ventricular (LV) function, hypertrophy, or geometric chamber distortion would be considered stage A, whereas patients who are asymptomatic but demonstrate LV hypertrophy and/or impaired LV function would be designated as stage B. Stage C then denotes patients with current or past symptoms of HF associated with underlying structural heart disease (the bulk of patients with HF), and stage D designates patients with truly refractory HF.

As used herein, the term "heart failure", in particular, refers to stages C and D of the ACC/AHA classification referred to above. In these stages, the subject shows typical symptoms of heart failure. Accordingly, a subject who suffers from heart failure, suffers from heart failure stage C or D according to the ACC/AHA classification.

The term "subject" as used herein relates to animals, preferably mammals, and, more preferably, humans. The subject to be tested, preferably, shall be suspected to suffer from heart failure.

However, it is envisaged that the subject does not suffer from cancer since it was shown that the CAIX level are be increased in cancer patients, in particular in patients suffering from metastatic cancer (see above). Furthermore, it is envisaged that the subject, preferably, does not suffer from peripheral artery disease, pulmonary hypertension, and/or pneumonia. Further, the subject, preferably, does not suffer from COPD (chronic obstructive pulmonary disease).

The term "cancer", as used herein, preferably refers to a proliferative disorder disease caused or characterized by the proliferation of cells which have lost susceptibility to normal growth control. The term, preferably, includes solid tumors arising in solid tissues or organs as well as hematopoietic tumors. The term encompasses tumors and any other proliferative disorders. Thus, the term is meant to include all pathological conditions involving malignant cells, irrespective of stage or of invasiveness. The cancer may be localized to a specific tissue or organ (e.g. in the breast, the prostate or the lung), and, thus, may not have spread beyond the tissue of origin. Furthermore, it is particularly contemplated that the cancer invasive, and, thus may have spread beyond the layer of tissue in which it originated into the normal surrounding tissues (frequently also referred to as locally advanced cancer). Invasive cancers may or not be metastatic. A cancer is metastatic, if it has spread from its original location to distant parts of the body. It is particularly contemplated that the term "cancer" refers to metastatic cancer. Accordingly, the subject in the context of the aforementioned method, preferably, does not suffer from metastatic cancer.

Moreover, it is also envisaged that the cancer is selected from the group consisting of cervical cancer, colorectal cancer, gastrointestinal cancer, leukaemia, lung cancer, mesothelioma, non-hodgkin's lymphoma, non-small cell lung cancer, ovarian cancer, pancreatic cancer, prostate cancer, skin cancer, small cell lung cancer, brain tumors, uterine cancer, head and neck tumors, liver cancer, renal cancer, neuroblastoma, intestine carcinoma such as rectum carcinoma, familiary adenomatous polyposis carcinoma and hereditary non-polyposis, esophageal carcinoma, labial carcinoma, larynx carcinoma, hypopharynx carcinoma, tong carcinoma, salivary gland carcinoma, adenocarcinoma, medullary thyroidea carcinoma, papillary thyroidea carcinoma, renal carcinoma, kidney parenchym carcinoma, ovarian carcinoma, cervical carcinoma, uterine corpus carcinoma, endometriuni carcinoma, chorion carcinoma, pancreatic carcinoma, testis carcinoma, urinary carcinoma, brain tumors such as glioblastoma, astrocytoma, meningioma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolid leukemia (AML), chronic myeloid leukemia (CML), adult T-cell leukemia lymphoma, hepatocellular carcinoma, gall bladder carcinoma, bronchial carcinoma, small cell lung carcinoma, multiple myeloma, basalioma, teratoma, retinoblastoma, choroidea melanoma, seminoma, rhabdomyosarcoma, craniopharyngeoma, osteosarcoma, chondrosarcoma, myosarcoma, liposarcoma, fibrosarcoma, Ewing sarcoma, plasmocytoma, Kaposi's sarcoma and melanoma.

The term "diagnosing" as used herein means assessing whether a subject as referred to in accordance with the method of the present invention suffers from heart failure, or not. As will be understood by those skilled in the art, such an assessment is usually not intended to be correct for 100% of the subj ects to be diagnosed. The term, however, requires that the assessment is correct for a statistically significant portion of the subjects (e.g. a cohort in a cohort study). Thus, the method of the present invention, however, at least provides an aid for establishing a final clinical diagnosis. Whether a portion is statistically significant, can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma, urine or serum and, most preferably, blood, plasma or serum. It is particularly preferred to determine the amount of CAIX in a serum sample. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

Carbonic anhydrase IX (herein also referred to as CAIX, frequently also referred to as carbonic anhydrase 9) is a polypeptide encoded by the Ca9 gene. It is an enzyme that catalyzes the rapid conversion of carbon dioxide and water to bicarbonate and protons, and vice versa (EC 4.2.1.1):

bicarbonate + H⁺<=> CO₂ + H₂O

CAIX is a transmembrane protein that comprises an N-terminal signal peptide, an extracellular proteoglycan domain and catalytic domain, a transmembrane segment, and a C-terminal tail. The polypeptide belongs to a large family of zinc metalloenzymes that catalyze the reversible hydration of carbon dioxide. CAIX is a transmembrane protein and the only tumor-associated carbonic anhydrase isoenzyme known. It was shown that it is expressed in all clear-cell renal cell carcinoma, but is not detected in normal kidney or most other normal tissues. The protein and the cDNA sequences of CAIX are, e.g., shown under GenBank Accession number NM_001216.2 GI:169636419.

CAIX as used herein, preferably, encompasses also variants of the aforementioned specific CAIX polypeptides. Such variants have at least the same essential biological and immunological properties as the specific CAIX polypeptides. In particular, they share the same essential biological and immunological properties if they are detectable by the same specific assays referred to in this specification, e.g., by ELISA assays using polyclonal or monoclonal antibodies specifically recognizing the said CAIX polypeptides. Preferably, the variant of CAIX has carbonic anhydrase activity (EC 4.2.1.1). Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific CAIX polypeptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific CAIX polypeptides or the aforementioned types of variants as long as these fragments have the essential immunological and biological properties as referred to above. Such fragments may be, e.g., degradation products of the CAIX polypeptides. Further included are variants which differ due to posttranslational modifications such as phosphorylation or myristylation.

Determining the amount of a peptide or polypeptide referred to in this specification relates to measuring the amount or concentration, preferably, semi-quantitatively or quantitatively. Measuring can be done directly or indirectly. Direct measuring relates to measuring the amount or concentration of the peptide or polypeptide based on a signal which is obtained from the peptide or polypeptide itself and the intensity of which directly correlates with the number of molecules of the peptide present in the sample. Such a signal - sometimes referred to herein as intensity signal -may be obtained, e.g., by measuring an intensity value of a specific physical or chemical property of the peptide or polypeptide. Indirect measuring includes measuring of a signal obtained from a secondary component (i.e. a component not being the peptide or polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay and methods which may utilize labelled molecules in various sandwich, competition, or other assay formats. Such assays are, preferably, based on detection agents such as antibodies which specifically recognize the peptide or polypeptide to be determined. The detection agents shall be either directly or indirectly capable of generating a signal indicating the presence or absence of the peptide or polypeptide. Moreover, the signal strength can, preferably, be correlated directly or indirectly (e.g. reverse- proportional) to the amount of polypeptide present in a sample. Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys™ analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a peptide or polypeptide comprises the steps of (a) contacting a cell capable of eliciting a cellular response the intensity of which is indicative of the amount of the peptide or polypeptide with the said peptide or polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the peptide or polypeptide.

Also preferably, determining the amount of a peptide or polypeptide comprises the step of measuring a specific intensity signal obtainable from the peptide or polypeptide in the sample. As described above, such a signal may be the signal intensity observed at an m/z variable specific for the peptide or polypeptide observed in mass spectra or a NMR spectrum specific for the peptide or polypeptide.

Determining the amount of a peptide or polypeptide may, preferably, comprises the steps of (a) contacting the peptide with a specific ligand, (b) (optionally) removing non-bound ligand, (c) measuring the amount of bound ligand. The bound ligand will generate an intensity signal. Binding according to the present invention includes both covalent and noncovalent binding. A ligand according to the present invention can be any compound, e.g., a peptide, polypeptide, nucleic acid, or small molecule, binding to the peptide or polypeptide described herein. Preferred ligands include antibodies, nucleic acids, peptides or polypeptides such as receptors or binding partners for the peptide or polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the peptide or polypeptide. Specific binding according to the present invention means that the ligand or agent should not bind substantially to ("cross-react" with) another peptide, polypeptide or substance present in the sample to be analyzed. Preferably, the specifically bound peptide or polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semi-quantitative or quantitative. Further suitable techniques for the determination of a polypeptide or peptide are described in the following.

First, binding of a ligand may be measured directly, e.g. by NMR or surface plasmon resonance. Second, if the ligand also serves as a substrate of an enzymatic activity of the peptide or polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/peptide or polypeptide" complex or the ligand which was bound by the peptide or polypeptide, respectively, may be contacted with a suitable substrate allowing detection by the generation of an intensity signal. For measurement of enzymatic reaction products, preferably the amount of substrate is saturating. The substrate may also be labelled with a detectable label prior to the reaction. Preferably, the sample is contacted with the substrate for an adequate period of time. An adequate period of time refers to the time necessary for an detectable, preferably measurable, amount of product to be produced. Instead of measuring the amount of product, the time necessary for appearance of a given (e.g. detectable) amount of product can be measured. Third, the ligand may be coupled covalently or non-covalently to a label allowing detection and measurement of the ligand. Labelling may be done by direct or indirect methods. Direct labelling involves coupling of the label directly (covalently or non-covalently) to the ligand. Indirect labelling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labelling or other detection methods as described above.

The amount of a peptide or polypeptide may be, also preferably, determined as follows: (a) contacting a solid support comprising a ligand for the peptide or polypeptide as specified above with a sample comprising the peptide or polypeptide and (b) measuring the amount peptide or polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labelled, carrying different ligands. Methods of producing such arrays, for example based on solid-phase chemistry and photo-labile protective groups, are generally known (US 5,744,305).

The term "amount" as used herein encompasses the absolute amount of a polypeptide or peptide, the relative amount or concentration of the said polypeptide or peptide as well as any value or parameter which correlates thereto or can be derived therefrom. Such values or parameters comprise intensity signal values from all specific physical or chemical properties obtained from the said peptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

The term "comparing" as used herein encompasses comparing the amount of the peptide or polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provides the desired assessment in a suitable output format, i.e. the diagnostic result. The said diagnostic result may, preferably, serve as an aid for establishing the final clinical diagnosis by, e.g., a medical practitioner.

Based on the comparison of the amount determined in step a) and the reference amount, it shall be possible to diagnose whether a subject suffers from heart failure, or not. Therefore, the reference amount is to be chosen so that either a difference or an identity in the compared amounts allows identifying those test subjects which belong into the group of subjects suffering from heart failure or into the group of subjects not suffering from heart failure. The method allows either excluding (rule-out) or identifying (rule-in) a subject as a subject suffering from heart failure. Differences in the amounts, i.e. increases or decreases, as used herein, preferably, are differences which are statistically significant. Whether a difference is statistically significant can be determined by the statistical techniques referred to elsewhere herein. Similarly, an identity in the amounts encompasses identical amounts and those differences in the amounts which are not statistically significant and which are within the standard deviations for a measured parameter.

The term "reference amount" as used herein refers to an amount which allows for allocation of a subject into either (i) the group of subjects suffering from heart failure or (ii) the group of subjects not suffering from heart failure. The reference amount applicable for an individual subject may vary depending on various physiological parameters such as age, gender, or subpopulation, as well as on the means used for the determination of the polypeptide or peptide referred to herein. A suitable reference amount may be determined from a reference sample to be analyzed together, i.e. simultaneously or subsequently, with the test sample.

Reference amounts can, in principle, be calculated for a cohort of subjects as specified above based on the average or mean values for a given biomarker by applying standard methods of statistics. In particular, accuracy of a test such as a method aiming to diagnose an event, or not, is best described by its receiver-operating characteristics (ROC) (see especially Zweig 1993, Clin. Chem. 39:561-577). The ROC graph is a plot of all of the sensitivity versus specificity pairs resulting from continuously varying the decision threshold over the entire range of data observed. The clinical performance of a diagnostic method depends on its accuracy, i.e. its ability to correctly allocate subjects to a certain prognosis or diagnosis. The ROC plot indicates the overlap between the two distributions by plotting the sensitivity versus 1-specificity for the complete range of thresholds suitable for making a distinction. On the y-axis is sensitivity, or the true-positive fraction, which is defined as the ratio of number of true-positive test results to the product of number of true-positive and number of false-negative test results. This has also been referred to as positivity in the presence of a disease or condition. It is calculated solely from the affected subgroup. On the x-axis is the false-positive fraction, or 1-specificity, which is defined as the ratio of number of false-positive results to the product of number of true-negative and number of false-positive results. It is an index of specificity and is calculated entirely from the unaffected subgroup. Because the true- and false-positive fractions are calculated entirely separately, by using the test results from two different subgroups, the ROC plot is independent of the prevalence of the event in the cohort. Each point on the ROC plot represents a sensitivity/-specificity pair corresponding to a particular decision threshold. A test with perfect discrimination (no overlap in the two distributions of results) has an ROC plot that passes through the upper left corner, where the true-positive fraction is 1.0, or 100% (perfect sensitivity), and the false-positive fraction is 0 (perfect specificity). The theoretical plot for a test with no discrimination (identical distributions of results for the two groups) is a 45° diagonal line from the lower left corner to the upper right corner. Most plots fall in between these two extremes. If the ROC plot falls completely below the 45° diagonal, this is easily remedied by reversing the criterion for "positivity" from "greater than" to "less than" or vice versa. Qualitatively, the closer the plot is to the upper left corner, the higher the overall accuracy of the test. Dependent on a desired confidence interval, a threshold can be derived from the ROC curve allowing for the diagnosis or prediction for a given event with a proper balance of sensitivity and specificity, respectively. Accordingly, the reference to be used for the aforementioned method of the present invention, i.e. a threshold which allows to discriminate between subjects suffering from heart failure or those which do not suffer from heart failure, preferably, by establishing a ROC for said cohort as described above and deriving a threshold amount therefrom. Dependent on a desired sensitivity and specificity for a diagnostic method, the ROC plot allows deriving suitable thresholds. It will be understood that an optimal sensitivity is desired for excluding a subject to suffer from heart failure (i.e. a rule out) whereas an optimal specificity is envisaged for a subject to be assessed as being at an increased risk (i.e. a rule in).

Preferably, said reference amounts are derived from a subject suffering from heart failure (or from a group thereof) and/or from a subject not suffering from heart failure (or from a group thereof). Preferably, the reference subject does not suffer from cancer.

If the reference amount is derived from a subject or a group of subjects known to suffer from heart failure, an essentially identical amount or an increased amount of CAIX in the test sample as compared to the reference amount is, preferably, indicative for heart failure.

If the reference is derived from a subj ect or a group of subj ects known not to suffer from heart failure, an essentially identical amount or a decreased amount of the CAIX in the test sample as compared to the reference amount, preferably, indicates that the subject does not suffer from heart failure.

Further, the reference amount may define a threshold amount, in particular a calculated reference amount, whereby an amount of CAIX in the sample of the test subject larger than the respective threshold shall be indicative for heart failure, while an amount of CAIX in the sample of the test subject lower than the respective threshold shall indicate that the subject does not suffer from heart failure.

Preferred reference amounts are indicated herein below:
A preferred reference amount indicating heart failure of about 52 pg/ml to about 98 pg/ml and, more preferably, about 60 to about 80 pg/ml, even more preferably of about 65 to 75 pg/ml. Even more preferably, the reference is an amount of about 52, or 60, or, most preferably 70 pg/ml. A test amount being essentially identical or increased shall be indicative for heart failure while a decreased amount in the test sample as compared to the reference amount shall indicate that the subject does not suffer from heart failure.
The term "about" as used herein means +/- 20%, +/- 10%, +/- 5%, +- 2 % or +-/ 1% from the specific values referred to.
In a preferred embodiment of the aforementioned method of the present invention, the method further comprises the step of recommending a therapy against heart failure, in case heart failure has been diagnosed by the method of the present invention.
The term "recommending" as used herein means establishing a proposal for a therapy which could be applied to the subject. However, it is to be understood that applying the actual therapy whatsoever is not comprised by the term. The therapy to be recommended depends on the outcome of the diagnosis provided by the method of the present invention.

Preferably, the therapy against heart failure is selected from administration of angiotensin-converting enzyme (ACE) inhibitors, administration of Angiotensin-II-receptor blocker (ARB), aldosteron antagonists, administration of beta blockers, administration of diuretics and Calcium antagonists.

The explanations and definitions given herein above apply mutatis mutandis to the following method of the present invention (except if stated otherwise)

### Method for assessing whether the myocardium in a subject suffering from heart failure is protected

Moreover, as already described elsewhere herein, it was shown by the inventors that CAIX is a marker for heart failure. Interestingly, however, CAIX levels are negatively correlated with Troponin levels in patients suffering from heart failure, whereas other cardiac markers (GDF-15 and NT-proBNP) showed a positive correlation to the CAIX level (see also Examples). The results observed in the studies carried out in the context of the present invention strongly indicate that significantly increased CAIX levels in patients may be associated with a better prognosis than less increased CAIX levels. In particular, the results indicate that CAIX may protect cardiomyocytes from necrosis.

Accordingly, the present invention relates to a method for assessing whether the myocardium in a subject suffering from heart failure is protected from necrosis, or not, comprising the step of a) determining the amount of CAIX in a sample from said subject.

In a preferred embodiment, the method further comprises the step b) of comparing the amount of CAIX to a reference. Based on this comparison step it is assessed whether the myocardium of the subject is protected from necrosis, or not.

In particular, it is assessed whether the myocardium of the subject suffering from heart failure is protected from necrosis, or not. This also includes partial protection of the myocardium from necrosis.

The term "necrosis" as used herein, preferably, refers to the formation necrotic tissue in a part/parts of the myocardium and, thus, to the formation of non non-viable myocardial tissue/myocytes. Preferably, the cell death occurs as a result of oxygen deprivation, which itself is caused by obstruction to the blood supply (ischemic necrosis). A result of necrosis, preferably, is the loss of the cell membrane and the release of proteolytic enzymes that cause cellular disruption. Necrosis in the context of the present invention, preferably, is localized. In it known in the art that necrotic cells are not functional and, thus, not contractile anymore, and therefore do not contribute to the pumping function of the heart. The function of necrotic cells can not be restored.

The subject to be tested in accordance with heart failure shall suffer from heart failure. Moreover, it is envisaged that the subject does not suffer from cancer, in particular, does not suffer from metastatic cancer. However, it is envisaged that the subject does not suffer from cancer since it was shown that the CAIX level may be increased in cancer patients (see above). Furthermore, it is envisaged that the subject, preferably, does not suffer from peripheral artery disease, pulmonary hypertension, and/or pneumonia. Further, the subject, preferably, does not suffer from COPD (chronic obstructive pulmonary disease).

The terms "sample" and "reference amount" have been defined herein elsewhere. The definitions apply accordingly.

The following are preferred algorithms for carrying out the diagnosis:
Preferably, the reference amount is derived from a subject or a group of subjects whose myocardium is known to be protected from necrosis. In this case, an essentially identical amount or an increased amount of a CAIX in the test sample as compared to the reference amount is, preferably, indicates that the myocardium of the test subject is protected from necrosis.

If the reference is derived from a subject or a group of subjects whose myocardium is known not to be protected from necrosis, an essentially identical amount or a decreased amount of the CAIX in the test sample as compared to the reference amount indicates that the myocardium of the test subject is not protected from necrosis.

Further, the present invention relates to the use of the CAIX polypeptide and/or of a detection agent, which specifically binds thereto in a sample of a subject for diagnosing heart failure.

Moreover, the present invention relates to the use of the CAIX polypeptide and/or of a detection agent, which specifically binds thereto in a sample of a subject for assessing whether the myocardium of a subject suffering from heart failure is protected from necrosis or not.

The term "detection agent" as used herein refers to an agent which is capable of specifically recognizing and binding the biomarker referred to herein (a cardiac Troponin or CAIX) when present in a sample. Moreover, said agent shall allow for direct or indirect detection of the complex formed by the said agent and the biomarker. Direct detection can be achieved by including into the agent a detectable label. Indirect labelling may be achieved by a further agent which specifically binds to the complex comprising the biomarker and the detection agent wherein the said further agent is than capable of generating a detectable signal. Suitable compounds which can be used as detection agents are well known in the art. Preferably, the detection agent is an antibody or aptamere which specifically binds to the biomarker. Antibodies as referred to herein include both polyclonal and monoclonal antibodies, as well as fragments thereof, such as Fv, Fab and F(ab)2 fragments that are capable of binding antigen or hapten. Also envisaged are single chain antibodies and humanized hybrid antibodies wherein amino acid sequences of a non-human donor antibody exhibiting a desired antigen-specificity are combined with sequences of a human acceptor antibody.

The present invention further relates to a device for diagnosing heart failure, said device comprising:
a) an analyzing unit comprising a detection agent for the CAIX polypeptide which allows for the determination of the amount of said CAIX polypeptide; and
b) an evaluation unit comprising a data processor having implemented an algorithm for comparing the amount determined by the analyzing unit with the reference amount stored in a database in order to establish the diagnosis of heart failure, wherein the reference amount is derived from a sample from a subject as described herein elsewhere in the context of the method for diagnosing heart failure, and the algorithm is an algorithm as set forth in the context of the said method.

The present invention further relates to a device for assessing whether the myocardium in a subject suffering from heart failure is protected from necrosis, said device comprising:
a) an analyzing unit comprising a detection agent for the CAIX polypeptide which allows for the determination of the amount of said CAIX polypeptide; and
b) an evaluation unit comprising a data processor having implemented an algorithm for comparing the amount determined by the analyzing unit with the reference amount stored in a database in order to establish the assessment whether the myocardium in a subject suffering from heart failure is protected from necrosis, wherein the reference amount is derived from a sample from a subject as described herein elsewhere in the context of the method assessing whether the myocardium in a subject suffering from heart failure is protected from necrosis, and the algorithm is an algorithm as set forth in the context of the said method.

The term "device" as used herein relates to a system comprising the aforementioned units operatively linked to each other as to allow the diagnosis according to the methods of the invention. Preferred detection agents which can be used for the analyzing unit are disclosed elsewhere herein. The analyzing unit, preferably, comprises said detection agents in immobilized form on a solid support which is to be contacted to the sample comprising the bio-markers the amount of which is to be determined. Moreover, the analyzing unit can also comprise a detector which determines the amount of detection agent which is specifically bound to the biomarker(s). The determined amount can be transmitted to the evaluation unit. Said evaluation unit comprises a data processing element, such as a computer, with an implemented algorithm for carrying out a comparison between the determined amount and a suitable reference. Suitable references can be derived from samples of subjects to be used for the generation of reference amounts as described elsewhere herein above. The diagnostic results may be given as output of parametric diagnostic raw data, preferably, as absolute or relative amounts. It is to be understood that these data may need interpretation by the clinician. However, also envisage are expert system devices wherein the output comprises processed diagnostic raw data the interpretation of which does not require a specialized clinician. Preferably, the device of the present invention can be used to carry out the aforementioned method of the present invention in an automated manner.

The present invention also encompasses a kit for diagnosing whether a subject suffers from heart failure, or not, said kit comprising at least a detection agent for the CAIX polypeptide and, preferably, standards which reflect the reference amounts as derived from a subject known to suffer from heart failure (or from a group thereof), or from a subject known not to suffer from heart failure (or from a group thereof).

The present invention encompasses a kit for assessing whether the myocardium of a subject suffering from heart failure is protected from necrosis, said kit comprising at least a detection agent for the CAIX polypeptide and, preferably, standards which reflect the reference amounts as derived from a subject whose myocardium is known to be protected from necrosis (or from a group thereof), or from a subject whose myocardium is known not to be protected from necrosis (or from a group thereof).

The term "kit" as used herein refers to a collection of the aforementioned components, preferably, provided in separately or within a single container. The container also comprises instructions for carrying out the method of the present invention. These instructions may be in the form of a manual or may be provided by a computer program code which is capable of carrying out the comparisons referred to in the methods of the present invention and to establish a diagnosis accordingly when implemented on a computer or a data processing device. The computer program code may be provided on a data storage medium or device such as a optical storage medium (e.g., a Compact Disc) or directly on a computer or data processing device. Moreover, the kit may, preferably, comprise standards which reflect the reference amounts as described and referred to elsewhere herein in detail. The detection agent is, preferably, immobilized on a carrier, and, preferably, a test stripe.

All references cited in this specification are herewith incorporated by reference with respect to their entire disclosure content and the disclosure content specifically mentioned in this specification.

The following Examples shall merely illustrate the invention. They shall not be construed, whatsoever, to limit the scope of the invention.

### Example 1: Determination of Troponin T, GDF-15, CAIX and NT-proBNP

Troponin T was determined using Roche's electrochemiluminescence ELISA sandwich test Elecsys Troponin T hs (high sensitive) STAT (Short Turn Around Time) assay. The test employs two monoclonal antibodies specifically directed against human cardiac Troponin T. The antibodies recognize two epitopes (amino acid position 125-131 and 136-147) located in the central part of the cardiac Troponin T protein, which consists of 288 amino acids (analytical sensitivity below 1,0).

NT-proBNP was determined using Roche's electrochemiluminescence ELISA sandwich test Elecsys proBNP II STAT (Short Turn Around Time) assay. The test employs two monoclonal antibodies which recognize epitopes located in the N-terminal part (1-76) of proBNP (1-108).

To determine the concentration of GDF-15 in serum and plasma samples, an Elecsys prototype test was employed, using a polyclonal, GDF-15 affinity chromatography-purified, goat anti-human GDF-15 IgG antibody from R&D Systems (AF957). In each experiment, a standard curve was generated with recombinant human GDF-15 from R&D Systems (957-GD/CF). The results with new batches or recombinant GDF-15 protein were tested in standard plasma samples and any deviation above 10% was corrected by introducing an adjustment factor for this assay. GDF-15 measurements in serum and plasma samples from the same patient yielded virtually identical results after correction for eventual dilution factors. The detection limit of the assay was 200 pg/ml.

CAIX was determined in serum samples using the Quantikine human CA IX(CA 9) ELISA from R&D systems (Minneapolis, MN, USA) according to the instructions of the manufacturer (Catalog Number DCA900). The ELISA consists of a sandwich format 96 well plate assay using a monoclonal-polyclonal /directly conjugated) format. The assay employs the quantitative sandwich enzyme immunoassay technique. A monoclonal antibody specific for CA9 has been pre-coated onto a microplate. Standards and samples are pipetted into the wells and any CA9 present is bound by the immobilized antibody. After washing away any unbound substances, an enzyme-linked polyclonal antibody specific for CA9 is added to the wells. Following a wash to remove any unbound antibody-enzyme reagent, a substrate solution is added to the wells and color develops in proportion to the amount of CA9 bound in the initial step. The color development is stopped and the intensity of the color is measured. The sensitivity of the assay is reported as 2.28 pg/ml with a standard curve up to 750 pg/ml.

Blood was centrifuged within 30 minutes and the resulting serum was kept at least - 20 degrees Celsius until tested.

### Example 2: Cohorts

149 clinically healthy human subjects were included into the study as controls, 52 males and 97 females (median age 41 years, range 19 to 56 years) These subjects had no cardiac disease as assessed by medical history and an electrocardiogram, no diabetes mellitus and no risk factors of these diseases. Moreover they had normal kidney function as assessed by normal creatinine values and malignant disorder.

In addition 42 patients with stable heart failure were included into the study. Stable heart failure was defined as no events within the past month, stable medication and change in body weight below 2 kg in the past month. They were 31 males and 11 females, median age 62 years (range 43 - 71 years). Heart failure was associated with a left ventricular ejection fraction below 40 %, cause of heart failure was identified as ischemic heart disease ( due to atheroscleroris of major heart vessels. They had normal kidney function and no evidence of a malignant disorder.

### Example 3: Results

When normal subjects were compared to patients with heart failure the following results were obtained (indicated are the median levels, for CAIX also the 25^{th} and 75^{th} are shown).

| | Healthy | HF |
|---|---|---|
| | N= 149 | n=42 |
| CAIX pg/ml | 39,5 | 76,4 |
| | 39,5 - 54,1 | 52,1 - 98,4 |
| NT-pro BNP pg/ml | 37,2 | 608 |
| GDF 15 pg/ml | 580 | 1873 |
| Troponin T pg/ml | neg | 11,7 |

The data obtained clearly indicate that CAIX levels in serum are higher in heart failure patients than in normal subjects suggesting a pathophysiological role of CAIX in non tumor disorders.

When the heart failure group was selected in patients above and below the median of CAIX the following results were obtained:

| | CAIX below | CAIX above |
|---|---|---|
| | Median | median |
| CAIX pg/ml | 51,87 | 98,41 |
| NT-pro BNP pg/ml | 595 | 690 |
| GDF 15 pg/ml | 1683 | 1916 |
| Troponin T pg/ml | 14,8 | 10,5 |

The table above illustrates interesting findings: increased CAIX levels were associated with increased markers of heart failure (NT-pro BNP and GDF 15). Interestingly, Troponin T levels were lower in the "high CAIX" group than in the "low CAIX" group. This indicates that CAIX similar to tumor diseases protects from cell death and may prolong survival.

### Conclusions:

CAIX has been shown to play a role in the progression of cancer as well in metastasis. The basis function of CAIX is its role in the protection of cancer cells from hypoxia and thus from cell death. In this context CAIX has been used as a biomarker of prognosis and tumor progression. In the present invention, surprisingly, CAIX has been found to be associated with heart failure and it is believed that it, may represent a natural defence mechanism in this disease. Even more surprisingly high levels of CAIX were associated with lower levels of Troponin T indicating that CAIX protects from cardiac necrosis in heart failure. Thus, increased CAIX levels may be beneficial in a heart failure patients.

## Claims

1. A method for diagnosing heart failure in a subject comprising determining the amount of carbonic anhydrase IX (CAIX) in a sample from the subject.

2. The method of claim 1, wherein the, thus, determined amount is compared to a reference amount, thereby diagnosing heart failure in said subject.

3. The method of claims 1 and 2, wherein the sample is blood, serum, or plasma.

4. The method of any one of claims 1 to 3, wherein the subject does not suffer from cancer, in particular from metastatic cancer.

5. The method of any one of claims 1 to 4, wherein the reference amount is derived from a reference subject known to suffer from heart failure.

6. The method of claim 5, wherein an essentially identical amount or an increased amount of CAIX in the test sample as compared to the reference amount indicates that the subject suffers from heart failure.

7. The method of any one of claims 1 to 4, wherein the reference amount is derived from a subj ect known not to suffer from heart failure.

8. The method of claim 7, wherein an essentially identical amount or a decreased amount of the CAIX in the test sample as compared to the reference amount indicates that the subject does not suffer from heart failure.

9. A method for assessing whether the myocardium in a subject suffering from heart failure is protected from necrosis, or not, comprising the step of determining the amount of CAIX in a sample from said subject.

10. The method of claim 9, further comprising the step of comparing the determined amount of CAIX to a reference amount, thereby assessing whether the myocardium of the subject is protected from necrosis.

11. The method of claims 9 and 10, wherein the sample is a blood, serum or plasma sample.

12. Use of the CAIX polypeptide and/or of a detection agent, which specifically binds thereto in a sample of a subject for diagnosing heart failure.

13. Use of the CAIX polypeptide and/or of a detection agent, which specifically binds thereto in a sample of a subject suffering from heart failure for assessing whether the myocardium of the subject suffering is protected from necrosis or not.

14. A device for diagnosing heart failure, said device comprising:
a) an analyzing unit comprising a detection agent for the CAIX polypeptide which allows for the determination of the amount of said CAIX polypeptide; and
b) an evaluation unit comprising a data processor having implemented an algorithm for comparing the amount determined by the analyzing unit with the reference amount(s) stored in a database in order to establish the diagnosis of heart failure, wherein the reference amount is derived from a sample from a subject(s) as defined in claim 5 and/or 7, and the algorithm is an algorithm as set forth claim 6 and/or 8.

15. A kit for diagnosing whether a subject suffers from heart failure, said kit comprising at least a detection agent for the CAIX polypeptide and standards which reflect the reference amounts as derived from a subject known to suffer from heart failure and/or from a subject known not to suffer from heart failure.
